Europäisches Patentamt

European Patent Office

Office européen des brevets

(11) Publication number:
**0 058 020**
**B1**

(12) **EUROPEAN PATENT SPECIFICATION**

(45) Date of publication of patent specification: 09.04.86

(51) Int. Cl.⁴: **A 61 B 1/04**

(21) Application number: **82300434.6**

(22) Date of filing: **28.01.82**

(54) **Endoscopes.**

(30) Priority: **03.02.81 JP 14545/81**

(43) Date of publication of application:
**18.08.82 Bulletin 82/33**

(45) Publication of the grant of the patent:
**09.04.86 Bulletin 86/15**

(84) Designated Contracting States:
**AT BE CH DE FR GB IT LI NL SE**

(56) References cited:
**DE-A-2 358 785**
**DE-A-2 923 490**
**DE-A-3 019 502**
**GB-A-1 486 681**
**JP-U-12 006 801**
**US-A-3 936 143**
**US-A-4 146 300**
**US-A-4 181 401**
**US-A-4 196 990**

**APPLIED OPTICS, vol. 16, no. 5, May 1977 NEW YORK (US) H. TSUCHIYA et al.: "Double eccentric connectors for optical fibers" pages 1323-1331**

(73) Proprietor: **OLYMPUS OPTICAL CO., LTD.**
**43-2, 2-chome, Hatagaya Shibuya-ku**
**Tokyo 151 (JP)**

(72) Inventor: **Shishido, Yoshio**
**No. 2-1-30-512, Fuchinobe Sagamihara-shi**
**Kanagawa-ken (JP)**

(74) Representative: **Kennedy, Victor Kenneth et al**
**G.F. REDFERN & CO. Marlborough Lodge 14**
**Farncombe Road**
**Worthing West Sussex BN11 2BT (GB)**

Courier Press, Leamington Spa, England.

# Description

This invention relates to endoscopes of the hard type, i.e. having a rigid casing, in which there is mounted an optical system to provide means for inspection or photographic recording of the interior of cavities, for example body cavities in the case of medical use, the endoscope having a relay lens system in the observing optical system, and an eye-piece cap which can be used to locate a photographic or television camera objective.

Normally, a camera objective is fitted with an adapter to engage and locate upon the eyepiece of an endoscope in order that a body cavity in which the front of the endoscope is inserted can be photographed for study and diagnosis, for example. However, if the optical axis of the endoscope optical system and the optical axis of the camera objective do not coincide, but are mutually off-set, then there will be some deflection of the image in the camera, which may lead to the loss of information at a marginal zone. Therefore, means may be provided on the camera to correct for any eccentricity of the optical axis of the endoscope. However, if such eccentricity is corrected by means provided on the camera, since it is to be expected that the eccentricity will be different for each individual endoscope, it will be necessary to readjust the eccentricity correction whenever the camera is fitted to the eyepiece of a different endoscope.

In order to avoid this disadvantage of misalignment between an optical axis and an eyepiece with a soft endoscope the Japanese Utility Model No. 126801/1980 proposes the displacement of the endoscope optical axis with respect to an endoscope eyepiece by use of an image guide fixing device for the eyepiece of a soft endoscope, in which the optical system for transmitting images to be observed is formed of a fibre-optic bundle, the above-mentioned eccentricity being forcibly corrected by displacement of the fixing device, for example, by a three-point locking screw grip, utilising the flexibility of the fibre bundle, and any consequential inclination of the end face of the bundle then being corrected by adjusting screws mounted in the fixing device. However, in a hard endoscope, in which images are transmitted by a rigid relay lens system, it is impossible to easily adjust the image transmitting optical system by force, as in the above-mentioned device disclosed in Japanese Utility Model No. 126801/1980.

An adapter device is disclosed in U.S. Patent No. 4 196 990, by which it is possible to correct for the above-mentioned eccentricity by moving a vision field mask in a falsely focussing position. However, if such a vision field mask is moved, in this way, the entire image may be so deflected from the optical axis of the endoscope eyepiece that the resolving power and picture quality will be reduced, which is most undesirable.

An object of the present invention, is to facilitate the taking of photographs with a hard endoscope using a removable camera fitted to the eyepiece part, by standardising the endoscope to correct for any eccentricity, using correcting means which mutually align the optical axis of the observing optical system and the optical axis of the camera, these correcting means being provided in the endoscope eyepiece structure and enabling a camera with a correctly centred adaptor to be used with any such endoscope with no alignment problems.

From one aspect the invention consists in a hard endoscope having a relay lens optical system to transmit an observed image to an eyepiece to which a camera may be fitted by means of an adaptor that is mounted on the camera to be concentric with the optical axis of the camera objective, characterised in that said eyepiece is provided with adjustable eccentricity correction means for effectively displacing the outer periphery of the eyepiece with respect to the endoscope optical axis and so standardise the endoscope by making the optical axis of the rigid-structured observing optical system of the hard endoscope substantially concentric with said outer periphery, whereby the optical axis of the fitted camera will be effectively aligned with the optical axis of the observing optical system of the hard endoscope.

Any eccentricity of the optical axis of the observing optical system of the hard endoscope with respect to the optical axis of the camera can then be easily controlled so as to be minimised or eliminated, without applying any undesirable force to the optical system of the endoscope.

The invention will now be described with reference to the drawings, in which:—

Figure 1 is a vertical section showing an eyepiece and associated operating part of one exemplary embodiment of an endoscope according to the present invention;

Figure 2 is a cross-section on line A—A of Figure 1;

Figure 3 is a schematic explanatory plan view showing the state of a photographic image in a case where the optical axis of the observing optical system of a hard endoscope and the optical axis of the photographing device side are not mutually aligned;

Figure 4 is a schematic explanatory plan view showing the state of a photographic image after the eccentricity shown in Figure 3 has been corrected;

Figure 5 is a partly sectional elevation showing part of the eyepiece part of a second exemplary embodiment of the present invention;

Figure 6 is a partly sectioned elevation showing part of the eyepiece of a third exemplary embodiment of the present invention;

Figure 7 is an explanatory plan view relating to the embodiment shown in Figure 6;

Figure 8 is a sectional view of the eyepiece of a fourth exemplary embodiment of the present invention;

Figure 9 is a cross-section on line B—B of Figure 8; and

Figure 10 is an explanatory cross-section relating to the embodiment shown in Figure 8.

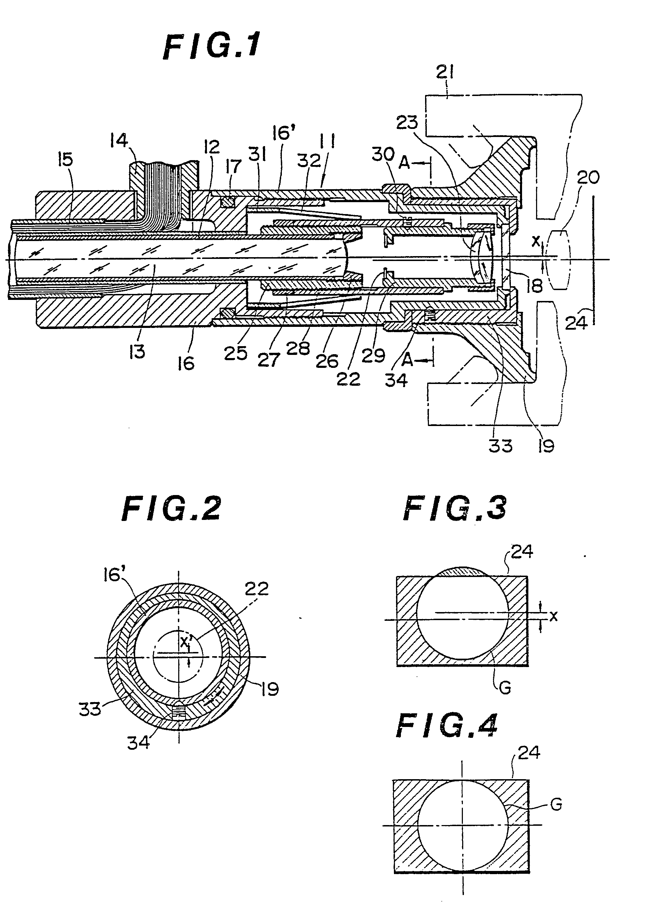

The first embodiment of the present invention will now be explained with reference to Figures 1 to 4. In these drawings, an operating part 11 of an endoscope contains a relay lens 13 fitted within a tube 12 and arranged to extend towards the rear of an input objective (not shown) at the front end of the inserted part of the endoscope, and a light guide 15 connected to a light source (not shown) via a coupling 14 and a flexible cable (not shown). The light guide 15 is fitted on the outer periphery of the tube 12 and it also extends to the front end of the inserted part of the endoscope. An outer tube 16' is screwed to the rear of the body 16 of the operating part 11 and sealed by an O-ring 17. An eyepiece frame 19 sealed by an eyepiece window 18 is screwed to the rear end of this outer tube 16'. The interior of the endoscope is filled with an inert gas, such as nitrogen, to prevent frosting of the optical system. A photographic camera having an objective lens 20 is removably fitted to the eyepiece frame 19 on the body 16 by an adapter 21 set on the camera with respect to the lens 20, so that, for example, an internal organ within the body cavity in which the inserted part of the endoscope is inserted can be illuminated via the light guide 15, and an image transmitted through the input objective (not illustrated) at the front end of the inserted part of the endoscope and passed by the relay lens 13 to be focused in the position of a vision field mask 22, and thus form a focused image on a film 24 in the case of a photographic camera, the image being fed out vie the eyepiece objective 23 and the camera lens 20.

The relay lens 13 is secured at its rear end by a lens fixing nut 26 screwed to the rear inner periphery of a fixing frame 25 that is soldered on the rear outer periphery of the tube 12. Further, a focusing frame 28 for the eyepiece objective 23 is screwed to this fixing frame 25 and fixed in position by a lock nut 27. A fitting frame 29 carries the vision field mask 22 at its front end and the objective 23 at its rear end, and is fixed to the focusing frame 28 by a clamping screw 30. An optical system holding part 31 having a male screw thread on its outer periphery at its front end is screwed onto a female screw thread formed on the inner periphery at the rear of the operating part body 16, this holding part 31 having tapered holding pieces 32 forming tongues extending inwardly and rearwardly. The focusing frame 28 is held by the inner periphery of these holding pieces 32, and is fixed with a binder or by solder so that the observing optical system is held securely fixed without applying any undesirable force.

In an endoscope having a rigid structure, using hard and inflexible optical elements, the fixed position of the observing optical system may be eccentric with respect to the periphery of the eyepiece, but the eccentricity cannot be corrected by the application of a transverse force, as in a soft endoscope using a fibre-optic bundle, and furthermore any alignment errors introduced by the assembly together of the focusing frame 28 fixed by the lock nut 27 of the focusing means, the eyepiece frame 29 and the photographic adapter 21 will be accumulative, so that in some cases the eccentricity $x$, that is, the deviation between the centre of the eyepiece frame 19 and the axis of the eyepiece lens 23 may reach 1 mm, for exmaple. If a camera is fitted to a hard endoscope having this eccentricity $x$ by means of an adapter 21, the deviation $x$ will be produced between the optical axis of the observing optical system of the hard endoscope and the optical axis of the camera, and, as shown in Figure 3 if the eccentricity $x$ is produced transverse to the longitudinal axis of a film 24 in a 35 mm camera, for example, the centre axis of an image G will be offset and lead to the loss of detail in the marginal zone that is shown shaded.

Therefore, in this embodiment of the invention, a rotatable eccentric centering frame 33 is fitted between the outer tube 16' and the eyepiece frame 19 as shown in Figure 2, so that the centering frame 33 can be rotated to a setting in which the eccentricity $x$ is substantially eliminated, and the image is central, on the longitudinal axis of the film stock, as the endoscope optical system axis is correctly positioned with respect to the centre of the outer peripheral circle of the centering frame 33, and the outer tube 16' and centering frame 33 can then be locked by a clamping screw 34. This construction thus enables such an eccentricity $x$ to be minimized or eliminated.

This centering adjustment may be carried out by using an eccentricity controlling jig (not illustrated) having a screen concentric with the outer peripheral cirle of the centering frame 33, and having crossed lines marking the centre position of the focusing lens.

After eliminating such an eccentricity or reducing it to a minimum, deviation between the centre of the eyepiece frame 19 and the centre of the vision field mask 22 and eyepiece 23 is effectively corrected, and if a photographing camera is fitted to the eyepiece frame 19 of the hard endoscope by an adapter 21, the deviation between the optical axis of the observing optical system of the endoscope and the optical axis of the camera will be minimised or eliminated, so that as shown in Figure 4, the axis of the film face 24 and the axis of the image G will coincide, or the image centre will lie on the longitudinal axis of the film, which will not lead to any loss of image area, even if the image centre and film centre do not coincide, and any cropping of the upper and lower edges, as drawn, will be the same.

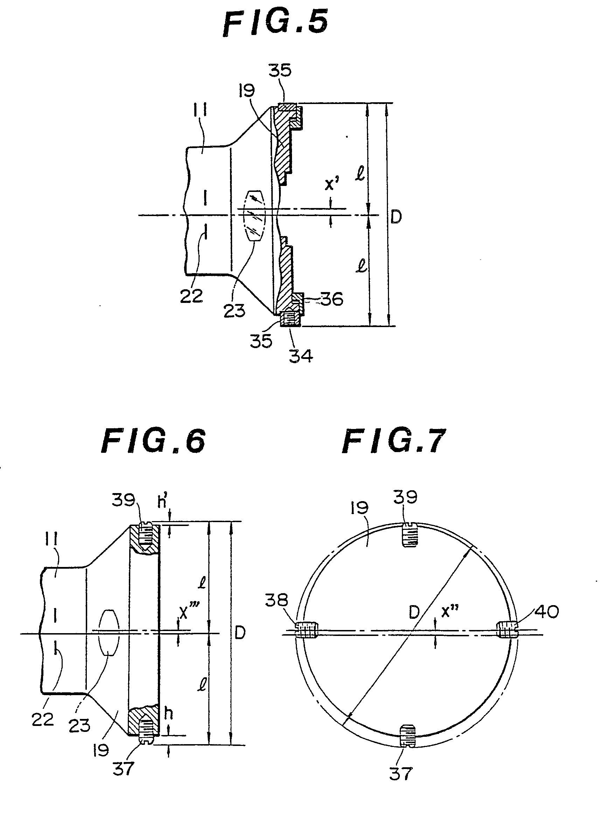

Figure 5 shows a second embodiment of the invention, in which an eccentric centering ring 35 is fitted on the outer pheriphery of the eyepiece frame 19, and may be rotated to adjust for correction of any eccentricity $x'$ so that the optical axis coincides with the centre of the eyepiece part, the fitting diameter D and radius $l$ being indicated here to emphasise that the eccentricity $x'$ can usually be completely corrected if a slight eccentricity in a direction normal to the eccentricity x' can be accepted. The ring 35 is held by a fixing frame 36 screwed to the eyepiece frame 19 so that the ring 35 can rotate, but not move away from the frame 19. The ring 35 is locked in position, when set, by a clamping screw 34.

By this formation, the eccentricity x' between the centre of the vision field mask 22, eyepiece 23, and eyepice frame 19 can be fully corrected by the proper rotational position of the centering ring 35. It may be advantageous to provide a key slot or flat on the adapter 21, to co-operate with a mating portion of the eyepiece frame 19, to identify the x-direction in either of the two embodiments described above.

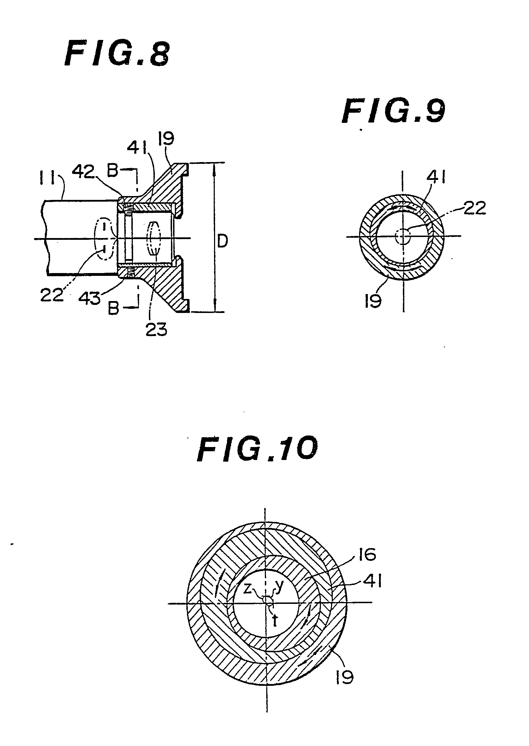

Figures 6 and 7 show a third embodiment of the invention, in which a set of eccentricity controlling screws are disposed around the periphery of the eyepiece frame 19. In the embodiment shown, there are four such screws, 37, 38, 39 and 40, with hemispherical heads, screwed to the outer periphery of the eyepiece frame 19, and individually adjusted to project by respective heights h, h', in such a manner that their heads define a fitting diameter D to absorb the eccentricity x''', and are bonded and fixed after this adjustment. Figure 6 shows the diameter D and radius *l*, to emphasise that adjustment may be effected only with respect to one direction, as described with reference to Figure 4. However, it will be apparent from a consideration of Figure 7 that the provision of individual screws enables correction to be completely effective for any direction of deviation.

Figures 8 to 10 show a fourth embodiment of the invention, in which an eccentric ring 41 similar to the ring 33 or 35 of the first and second embodiments is provided, together with an eccentric eyepiece frame 19. Respective clamping screws 42 and 43 are provided. With this modification any eccentricity T between the image centre, or centre of the vision field mask 22, and the centre of the operating part body 16, any eccentricity Y between the operating part body 16 and the centering ring 41, and eccentricity Z between the centering ring 41 and the eyepiece frame 19 can be fully eliminated if $T + Y \geqq Z$, as it will then be possible to reduce all eccentricity to zero by adjusting the centre of the fitting diameter D of the eyepiece part to which the adapter is fitted and the image centre of the vision field mask 22 by rotating the respective eccentric rings. As there is only one centering means provided in the first and second embodiments, the eccentricity can be minimised but may not be reduced to zero in any direction of deviation, as has been described with reference to Figure 4, as it will become zero only when $T = Y$.

It should be emphasised that 35 mm. film cameras, 16 mm. cinematic cameras, and television cameras can be used with suitably designed adapters to fit on the eyepiece frame.

## Claims

1. A hard endoscope having a relay lens optical system (13) to transmit an observed image to an eyepiece (19) to which a camera may be fitted by means of an adaptor (21) that is mounted on the camera to be concentric with the optical axis of the camera objective, characterised in that said eyepiece (19) is provided with adjustable eccentricity correction means (16', 19, 33; 19, 35, 36; 37—40; 16, 19, 41) for effectively displacing the outer periphery of the eyepiece with respect to the endoscope optical axis and so standardise the endoscope by making the optical axis of the rigid-structured observing optical system of the hard endoscope substantially concentric with said outer periphery, whereby the optical axis of the fitted camera will be effectively aligned with the optical axis of the observing optical system of the hard endoscope.

2. A hard endoscope according to Claim 1, characterised in that said eccentricity correcting means comprises an eccentric centering ring (33) rotatable between an inner peripheral member (16') and the outer periphery (19) of the eyepiece which engages the adaptor of a fitted camera, and clamping means (34) for securing the eccentric ring, when adjusted.

3. A hard endoscope according to Claim 1, characterised in that said eccentricity correcting means comprises an eccentric ring (35) rotatable about the outer periphery (19) of the eyepiece, and clamping means (34) for securing the eccentric ring, when adjusted.

4. A hard endoscope according to Claim 1, characterised in that said eccentricity correcting means comprises a plurality of adjustable projections (37—40) disposed evenly around the periphery of the eyepiece.

5. A hard endoscope according to Claim 1, characterised in that said eccentricity correcting means comprises an eccentric ring (41) rotatably fitted to the inner periphery of an eccentric eyepiece ring (19), and respective clamping means (42, 43) for securing each said eccentric ring, when adjusted.

## Patentansprüche

1. Hartes Endoskop mit einem übertragenden optischen Linsensystem (13) zum Übermitteln eines beobachteten Bildes an ein Okular (19), an das eine Kamera mittels eines Adapters (21) angesetzt werden kann, der so an der Kamera befestigt ist, daß dessen optische Achse mit der des Kameraobjektivs konzentrisch ist, dadurch gekennzeichnet, daß das Okular (19) mit einstellbaren, die Exzentrizität korrigierenden Mitteln (16', 19, 33; 19, 35, 36; 37—40; 16, 19, 41) versehen ist, um wirksam die äußere Peripherie des Okulares in bezug auf die optische Achse des Endoskops zu versetzen, damit das Endoskop normiert werden kann, und zwar dadurch, daß die optische Achse des fest ausgebildeten optischen Beobachtungssystems des harten Endoskops im wesentlichen konzentrisch mit der äußeren Peripherie ausgerichtet werden kann, wobei dadurch die optische Achse der angesetzten Kamera wirksam mit der optischen Achse des betrachtenden optischen Systems des harten Endoskopes ausgerichtet werden kann.

2. Hartes Endoskop nach Anspruch 1, dadurch gekennzeichnet, daß die die Exzentrizität korrigierenden Mittel einen exzentrischen Zentrierring (33) aufweisen, der drehbar zwischen einem inneren peripheren Teil (16') und der äußeren Peripherie (19) des Okulars angeordnet ist und der mit dem Adapter der angesetzten Kamera in Eingriff steht und daß Klemmmittel (34) vorgesehen sind zur Sicherung des Exzenterringes, wenn er eingestellt ist.

3. Hartes Endoskop nach Anspruch 1, dadurch gekennzeichnet, daß die die Exzentrizität korrigierenden Mittel einen exzentrischen Ring (35) enthalten, der um die äußere Peripherie (19) des Okulares drehbar ist und daß Klemmmittel (34) vorgesehen sind, um den Exzentterring zu sichern, wenn er eingestellt worden ist.

4. Hartes Endoskop nach Anspruch 1, dadurch gekennzeichnet, daß die die Exzentrizität korrigierenden Mittel eine Mehrzahl von einstellbaren Vorsprüngen (37—40) aufweisen, die gleichmäßig um die Peripherie des Okulares verteilt sind.

5. Hartes Endoskop nach Anspruch 1, dadurch gekennzeichnet, daß die die Exzentrizität korrigierenden Mittel einen Exzenterring (41) aufweisen, der drehbar zu der inneren Peripherie eines exzentrischen Okularringes (19) befestigt ist und daß entsprechende, Klemmmittel (42, 43) vorgesehen sind, um den Exzenterring zu sichern, wenn er eingestellt worden ist.

### Revendications

1. Endoscope rigide comportant un système optique (13) à lentille de relais servant à transmettre une image observée à un oculaire (19) sur lequel un appareil de prise de vues peut être monté à l'aide d'un adaptateur (21) et qui est monté sur l'appareil de prise de vues de manière à être concentrique à l'axe optique de l'objectif de l'appareil de prise de vues, caractérisé en ce que ledit oculaire (19) est muni de moyens réglables de correction de l'excentricité (16', 19, 33; 19, 35, 36; 37—40; 16, 19; 41) pour déplacer de façon effective le pourtour extérieur de l'oculaire par rapport à l'axe optique de l'endoscope et à standardiser l'endoscope en rendant l'axe optique du système optique d'observation à structure rigide de l'endoscope rigide sensiblement concentrique par rapport audit pourtour extérieur, ce qui a pour effet que l'axe optique de l'appareil de prise de vues monté peut être aligné de façon effective avec l'axe optique du système optique d'observation de l'endoscope rigide.

2. Endoscope rigide selon la revendication 1, caractérisé en ce que lesdits moyens de correction de l'excentricité comportent une bague de centrage excentrique (33) pouvant tourner entre un organe périphérique intérieur (16') et le pourtour extérieur (19) de l'oculaire qui contacte l'adaptateur d'un appareil de prise de vues monté, et des moyens de serrage (34) servant à bloquer la bague excentrique, lorsqu'elle est réglée.

3. Endoscope rigide selon la revendication 1, caractérisé en ce que lesdits moyens de correction de l'excentricité comportent une bague excentrique (35) pouvant tourner autour du pourtour extérieur (19) de l'oculaire, et des moyens de serrage (34) servant à bloquer la bague excentrique, lorsqu'elle est réglée.

4. Endoscope rigide selon la revendication 1, caractérisé en ce que lesdits moyens de correction de l'excentricité comportent une pluralité de parties saillantes réglables (37—40) réparties régulièrement sur le pourtour de l'oculaire.

5. Endoscope rigide selon la revendication 1, caractérisé en ce que lesdits moyens de correction de l'excentricité comportent une bague excentrique (41) montée de façon à pouvoir tourner sur le pourtour intérieur d'une bague excentrique (19) de l'oculaire, et des moyens de serrage respectifs (42, 43) servant à bloquer chacune desdites bagues excentriques lorsqu'elles sont réglées.

# FIG.1

# FIG.2

# FIG.3

# FIG.4

1

# FIG.5

# FIG.6

# FIG.7

# FIG.8

# FIG.9

# FIG.10